(19) Europäisches Patentamt / European Patent Office / Office européen des brevets

(11) **EP 1 924 689 B1**

(12) **EUROPEAN PATENT SPECIFICATION**

(45) Date of publication and mention of the grant of the patent:
**13.08.2014 Bulletin 2014/33**

(51) Int Cl.:
**C12N 9/64** (2006.01)     **C07K 14/745** (2006.01)

(21) Application number: **06806746.1**

(22) Date of filing: **01.09.2006**

(86) International application number:
**PCT/EP2006/065930**

(87) International publication number:
**WO 2007/026021 (08.03.2007 Gazette 2007/10)**

(54) **HYDROPHOBIC INTERACTION CHROMATOGRAPHY PURIFICATION OF FACTOR VII POLYPEPTIDES**

REINIGUNG VON FAKTOR-VII-POLYPEPTIDEN MITTELS HYDROPHOBER INTERAKTIONSCHROMATOGRAFIE

PURIFICATION DE POLYPEPTIDES DU FACTEUR VII PAR CHROMATOGRAPHIE D'INTERACTION HYDROPHOBE

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.09.2005 EP 05107990**

(43) Date of publication of application:
**28.05.2008 Bulletin 2008/22**

(73) Proprietor: **Novo Nordisk Health Care AG**
**Thurgauerstrasse 36/38**
**8050 Zürich (CH)**

(72) Inventor: **KRARUP, Janus**
**DK-2820 Gentofte (DK)**

(74) Representative: **Nilsson, Karin Norvin**
**Novo Nordisk A/S**
**Corporate Patents**
**Novo Allé**
**2880 Bagsværd (DK)**

(56) References cited:
**WO-A-2005/111225     US-B1- 6 777 390**

- **SOENDERKAER SUSANNE ET AL: "Effects of sucrose on rFVIIa aggregation and methionine oxidation." EUROPEAN JOURNAL OF PHARMACEUTICAL SCIENCES : OFFICIAL JOURNAL OF THE EUROPEAN FEDERATION FOR PHARMACEUTICAL SCIENCES. APR 2004, vol. 21, no. 5, April 2004 (2004-04), pages 597-606, XP002407467 ISSN: 0928-0987**
- **BOLT GERT ET AL: "Posttranslational N-glycosylation takes place during the normal processing of human coagulation factor VII" GLYCOBIOLOGY, vol. 15, no. 5, May 2005 (2005-05), pages 541-547, XP002407469 ISSN: 0959-6658**
- **BJOERN S ET AL: "HUMAN PLASMA AND RECOMBINANT FACTOR VII CHARACTERIZATION OF O GLYCOSYLATIONS AT SERINE RESIDUES 52 AND 60 AND EFFECTS OF SITE-DIRECTED MUTAGENESIS OF SERINE 52 TO ALANINE" JOURNAL OF BIOLOGICAL CHEMISTRY, vol. 266, no. 17, 1991, pages 11051-11057, XP002407468 ISSN: 0021-9258**
- **JOSIC D ET AL: "Preparation of vitamin K-dependent proteins, such as clotting factors II, VII, IX and X and clotting inhibitor Protein C" JOURNAL OF CHROMATOGRAPHY B: BIOMEDICAL SCIENCES & APPLICATIONS, ELSEVIER, AMSTERDAM, NL, vol. 790, no. 1-2, 25 June 2003 (2003-06-25), pages 183-197, XP004426799 ISSN: 1570-0232**

• DATABASE MEDLINE [Online] US NATIONAL LIBRARY OF MEDICINE (NLM), BETHESDA, MD, US; September 1975 (1975-09), COOPER H A ET AL: "Effects of thrombin treatment of preparations of factor VIII and the Ca2+-dissociated small active fragment." XP002360538 Database accession no. NLM1080490 & THE JOURNAL OF CLINICAL INVESTIGATION. SEP 1975, vol. 56, no. 3, September 1975 (1975-09), pages 751-760, ISSN: 0021-9738

**Description**

FIELD OF THE INVENTION

The present invention relates to a process for the hydrophobic interaction chromatography (HIC) purification of Factor VII polypeptides.

BACKGROUND OF THE INVENTION

The proteins involved in the clotting cascade, including, e.g., Factor VII, Factor VIII, Factor IX, Factor X, and Protein C, are proving to be useful therapeutic agents to treat a variety of pathological conditions. Accordingly, there is an increasing need for formulations comprising these proteins that are pharmaceutically acceptable and exhibit a uniform and predetermined clinical efficacy. The overall industrial-scale process for the purification of drug substances of a Factor VII polypeptide may in certain instances suffer from the drawback that the drug substance comprises a considerable amount of product-related (Factor VII-related) impurities (such as impurities contained in late eluting peaks (including glyco-variants with differing level of N-linked glycosylation ("des-N-glycan forms")), oxidized forms, proteolytically degraded forms (heavy-chain cleaved forms), or aggregates having a higher molecular weight than the Factor VII polypeptide of interest). This is - of course - undesirable and in some instances also unacceptable.

The article "Amino acid sequence and posttranslational modifications of human factor VIIa from plasma and transfected baby hamster kidney cells", Biochemistry, 1988 Oct 4; 27(20):7785-93, reports that some heavy- chain degradation products co-purify with intact activated Factor VII.

The article "FVIIa derivatives obtained by autolytic and controlled cathepsin G mediated cleavage". FEBS Lett. 1993 Feb 15; 317(3):245-9, states that heavy-chain cleaved forms of Factor VII cannot be isolated from Factor VII under non-denaturing conditions.

US 6,777,390 (Baxter) concerns purification of factor VII from cryosupernatant by AIEX and subsequent hydrophobic chromatography on Phenyl-Sepharose. The described pharmaceutical factor VII preparations contain less than 5% activated factor VII (Factor VIIa).

Soenderkaer et al. describes the effects of sucrose on rFVIIa aggregation and methionine oxidation; assays for analysing content of aggregates and oxidized forms are described.

Bolt et al. describes that post-translational N-glycosylation takes place during the normal processing of human coagulation Factor VII.

Bjoern et al. is directed to characterization of plasma- and recombinant FVIIa O-glycosylation at serine residues 52 and 60 and the effects of site-directed mutagenesis of serine-52 to alanine

To the present inventors' knowledge, the problem of removal of product-related impurities in a drug substance of a FVII polypeptide in industrial-scale processes has not been addressed in the prior art.

BRIEF DESCRIPTION OF THE INVENTION

The present invention provides a process for reducing the content of forms of Factor VII lacking one or more N-linked glycan(s) in a drug substance of a recombinantly made activated FVII polypeptide, said process comprising the steps of:

(a) contacting the drug substance with a hydrophobic interaction chromatography material under conditions which facilitate binding of a portion of said drug substance to said hydrophobic interaction chromatography material, said drug substance comprising a salt selected from the list of: ammonium acetate, ammonium sulphate, ammonium chloride, sodium chloride, sodium acetate, sodium sulphate, potassium acetate, potassium chloride, and potassium sulphate, and/or a zwitterion selected from the list of: glycine, alanine, beta-alanine, leucine, and isoleucine, in a concentration of in the range of 0.0-0.1 M or in the range of 0.5 M to 85% of the saturation concentration for the respective salt at the temperature at which step (a) is carried out;

(b) optionally washing said hydrophobic interaction chromatography material with a washing buffer; and

(c) eluting said hydrophobic interaction chromatography material with an elution buffer, and collecting a purified drug substance of the activated Factor VII polypeptide as an eluate;

wherein the content of forms of Factor VII polypeptide lacking one or more N-linked glycan(s) in the purified drug substance collected in step (c), as determined by the methods defined herein, is reduced with at least 50% (w/w) compared to the content contained in the drug substance applied in step (a).

BRIEF DESCRIPTION OF THE FIGURES

Figure 1 is a chromatogram of rhFVII separation on TSK-Gel phenyl 5PW, cf. Example 1.

Figure 2 is a chromatogram of FVIIa analogue separation on TSK Phenyl 5PW, cf. Example 3.

Figure 3 illustrates elution of impurities throughout the product peak, cf. Example 3.

Figure 4 illustrates an SDS-PAGE of HIC load showing high-molecular weight compounds at 185, 140 and 90 kDa.

Figure 5 illustrates an SDS-PAGE of HIC pool showing that the high-molecular weight compounds at 185, 140 and 90 kDa have almost been eliminated.

Figure 6 is a chromatogram of rhFVII separation on TSK-Gel phenyl 5PW, cf. Example 6.

Figure 7 is an SDS-PAGE gel illustrating the partial removal of GD-FVII. Lane 1: mw standard, lane 2: load sample, lane 3: leading edge, lane 4: main peak, lane 5: trailing edge, cf. Example 6.

Figure 8 illustrates a HPLC chromatogram of FVII and its product-related impurities.

DETAILED DESCRIPTION OF THE INVENTION

The present inventors have now found that by following a particular hydrophobic interaction chromatography procedure wherein a particular salt and/or zwitterion is used, it is possible to reduce, or virtually eliminate, the presence of impurities such as "late eluting peaks" comprising N-glycovariants without N-glycans in positions Asn145 and/or Asn322 (of human FVII) can be reduced.

The present invention provides a process for reducing the content of forms of Factor VII lacking one or more N-linked glycans in a drug substance of a recombinantly made activated Factor VII polypeptide, using hydrophobic interaction chromatography, cf. claim 1.

The process is, thus, particularly relevant for drug substances of Factor VII polypeptides that initially have a considerable content of Late eluting peaks, namely a pool of Late eluting peaks of at least 2% (w/w) of the total amount of Factor VII polypeptides. It should be understood that, in some instances, industrial-scale drug substances of Factor VII polypeptides may include even higher amounts of Late eluting peaks, e.g. at least 3%, such as at least 4%, or at least 5%, some times up to about 10-12%, of Late eluting peaks, and that the process of the present invention is even more relevant for such drug substances. Such drug substances may be obtained directly from fermentation processes, but more usually as the result of initial crude product purification.

The process is furthermore relevant for drug substances of Factor VII polypeptides that initially have a considerable content of oxidized forms, proteolytically degraded forms, and/or aggregates having a higher molecular weight than the Factor VII polypeptide of interest, namely a pool of proteolytically degraded forms of at least 2% (w/w) of the total amount of Factor VII polypeptides and/or oxidized forms of at least 1% (w/w) of the total amount of Factor VII polypeptides.

When used in conjunction with Factor VII polypeptides, the percentage (%) of product-related impurities (e.g. Late eluting peaks) is stated as a percentage by weight of the total content of protein (product + product-related impurities). Thus, a two-fold reduction of product-related impurities (e.g. Late eluting peaks), e.g. from 2% to 1%, constitutes a relative reduction of 50%; a reduction from, e.g., 8% to 2% thus designates a four-fold reduction, i.e. a relative reduction of 75% ; and a reduction from 12% to 6% constitutes a two-fold reduction of content of product-related impurities (i.e. 50% reduction) and *not* a reduction of 6%-points.

The term "Product-related impurities" include, without limitation, glycovariants with differing level of N-linked glycosylation (including Factor VII polypeptides lacking one or more N-linked glycans e.g., in positions Asn145 and/or Asn322 of human FVII), oxidized forms, proteolytically degraded forms (auto-catalyzed degradation of the heavy chain of the molecule) and aggregates having a higher molecular weight than the Factor VII polypeptide of interest (including dimers, oligomers, polymers).

Although the exact chemical constitution of late eluting peak-impurities remains partly unknown, the expression "Late eluting peaks" is intended to mean Factor VII polypeptide-related structures having a higher relative retention time than the Factor VII polypeptide of interest, possibly undesirable glycoforms such as forms where the Factor VII polypeptide is lacking one or more (e.g. one or both) N-linked glycans.

The "relative retention" of the "Late eluting peaks" (relative to the FVII polypeptide of interest, e.g. rFVIIa) is determined using a RP-HPLC assay as described in *General methods,* below.

For quantification purposes, the relative retention time (RR) for such "Late eluting peaks" is typically from 1.040 to 1.300 (the rFVIIa main peak being at 1.000) as determined by the RP-RPLC analytical method.

The content of Late eluting peaks in a drug substance of a Factor VII polypeptide can be determined as described in *General methods,* below.

The content of oxidized forms, proteolytically degraded forms, and high molecular weight forms (dimers, oligomers, aggregates) of a Factor VII polypeptide in a drug substance of said polypeptide can be determined as described in *General methods,* below.

Although not limited thereto, the process of the present invention is particularly feasible for "industrial-scale" (or "large-scale") drug substances of a Factor VII polypeptide. By the term "industrial-scale" is typically meant methods wherein the volume of liquid Factor VII polypeptide compositions is at least 10 L, such as at least 50 L, e.g. at least 500 L, or at least 5000 L, or where the weight of the product is at least 1 g (dry matter), such as at least 10 g, e.g. at least 50 g, e.g. 1-1000 g.

The expression "drug substance" used herein is intended to mean a solid mass as well as a liquid mass, e.g. a solution or suspension comprising the Factor VII polypeptide. The expression "drug substance" is in particular meant to refer to a "large" volume or mass, i.e. referring to volumes and masses known from large-scale and industrial-scale processes.

The term "Factor VII polypeptide" is defined further below.

The expression "salt" used herein is intended to refer to one or more salts capable of making the Factor VII polypeptide so relatively hydrophobic that is will bind to the hydrophobic interaction chromatography material. Preferred examples of salts are those selected from combinations of particular cations and particular anions. The group of cations comprises ammonium, sodium and potassium, and the group of anions comprises sulfate, acetate and chloride. Examples of salts are ammonium acetate, ammonium sulphate, ammonium chloride, sodium chloride, sodium acetate, sodium sulphate, potassium acetate, potassium chloride and potassium sulphate. Preferred salts are ammonium acetate, ammonium sulphate, sodium acetate, and sodium chloride. Another group of preferred salts contains ammonium acetate, ammonium sulphate, and sodium acetate.

In the following table is stated the solubility (saturation) at 20°C and the molar concentration corresponding to 85% of saturation for a series of useful salts.

| Salt | Molecular weight (g/mol) | Solubility (g/100 g water) | Solubility mol/L | Solubility 85% |
|---|---|---|---|---|
| Ammonium acetate | 77.1 | 148.0 | 19.2 | 16.3 |
| Ammonium chloride | 53.5 | 39.5 | 7.4 | 6.3 |
| Ammonium sulfate | 132.1 | 76.4 | 5.8 | 4.9 |
| Calcium acetate | 158.2 | 43.6 | 2.8 | 2.3 |
| Calcium chloride | 110.9 | 81.3 | 7.3 | 6.2 |
| Lithium acetate | 66.0 | 45.0 | 6.8 | 5.8 |
| Lithium chloride | 42.4 | 84.5 | 19.9 | 16.9 |
| Magnesium choride | 95.2 | 56.0 | 5.9 | 5.0 |
| Manganese(II) chloride | 125.8 | 77.3 | 6.1 | 5.2 |
| Potassium acetate | 98.1 | 269.0 | 27.4 | 23.3 |
| Potassium chloride | 74.6 | 35.5 | 4.8 | 4.0 |
| Potassium phosphate | 212.3 | 106.0 | 5.0 | 4.2 |
| Sodium acetate | 82.0 | 50.4 | 6.1 | 5.2 |
| Sodium chloride | 58.4 | 36.0 | 6.2 | 5.2 |
| Sodium citrate | 294.1 | 72.0 | 2.4 | 2.1 |
| Sodium sulfate | 142.0 | 28.1 | 2.0 | 1.7 |
| Glycine | 75.0 | 22.5 | 3.0 | 2.6 |

Above data at 20°C

The expression "zwitterions" is intended to mean an ion which carries both a negative and a positive electrical charge, but which forms a neutral molecule. Examples of useful zwitterions are neutral amino acids, e.g. glycine, alanine, beta-alanine, leucine, isoleucine, etc., in particular glycine and beta-alanine.

*Step (a) - Contacting the drug substance with a hydrophobic interaction chromatography material*

In a first step of the process, the drug substance of the Factor VII polypeptide is contacted with a hydrophobic interaction chromatography material under conditions which facilitate binding of a portion of said drug substance to said hydrophobic interaction chromatography material. The drug substance should comprise a salt and/or a zwitterion in a concentration of in the range of 0.0-0.1 M or in the range of 0.5 M to 85% of the saturation concentration for the respective salt (at the temperature of the loading solution). The aim is to facilitate binding of a portion of said drug substance of the Factor VII polypeptide to said hydrophobic interaction chromatography material. In a particularly preferred embodiment, the drug substance comprises a salt in a concentration of in the range of 0.5 M to 85% of the saturation concentration, e.g. 0.7-2.2 M.

It will be understood that the saturation concentration of a particular salt (and thereby the concentration which constitutes 85% of the saturation concentration) will vary depending on the actual temperature used, e.g. the saturation concentration will normally be lower at 5°C compared to that of 20°C. The solubility (saturation) at 20°C of particular, useful salts are described above. Solubility at 20°C for other salts as well as solubility at other temperatures (e.g. 5°C) can be found in general handbooks of chemistry, such as, e.g. The CRC Handbook of Chemistry and Physics (CRC Press). Alternatively, solubility curves for dissolution of a particular solid (salt) in water can easily be obtained by dissolving a known weight of a salt in varying, known volumes of water, and at each concentration allowing the solution to cool to determine at which temperature the salt begins to crystallize out of solution. That is, for a certain weight of salt, the quantity of water is progressively increased, and for each increase the temperature required to reach the point of saturation is determined. The weight of salt and the weight of water for each saturation temperature represent the concentration of a saturated solution for that temperature, and is expressed in terms of g salt per 100 g water. A solubility curve is then drawn by plotting the g salt per 100 g water on $\gamma$-axis against saturation temperature on x axis. As such, the solubility of a solid may be expressed as the number of moles of solid dissolved in a liter of liquid, or as the mole fraction of the solid, or, as in this experiment, as the number of grams of solid dissolved in 100 mL of liquid.

It is to be understood that the term "the range of 0.5 M to 85% of the saturation concentration for the respective salt" is intended to mean a range from 0.5 M to 85% of the saturation concentration of a particular salt at the actual temperature at which the relevant method step or steps (loading (a), washing (b), elution (c)) is/are carried out.

By the term "portion" in connection with step (a) is meant at least 30% (i.e. 30-100%) of the mass of the Factor VII polypeptide present in the drug substance of the Factor VII polypeptide. It should be understood that it in most instances is desirable to bind far more than 30% of the mass of the Factor VII polypeptides, e.g. at least 50%, or at least 70%, or a predominant portion. By the term "predominant portion" is meant at least 90% of the mass of the Factor VII polypeptide present in the drug substance of the Factor VII polypeptide. Preferably an even higher portion becomes bound to the hydrophobic interaction chromatography material, e.g. at least 95% of the mass, or at least 98% of the mass, or at least 99% of the mass, or even substantially all of the mass of the Factor VII polypeptide present in the drug substance of the Factor VII polypeptide.

The drug substance of the Factor VII polypeptide typically originates from an industrial-scale production process, e.g. a cell culture, a microbial process, a cloned animal (e.g. cows, pigs, sheep, goats, and fish) or insect, or the like, in particular from a cell culture.

The hydrophobic interaction chromatography material is a matrix substituted with hydrophobic ligands such as ethyl, butyl, phenyl or hexyl (which appears to be responsible for binding the protein). Preferred materials are those substituted with butyl and/or phenyl ligands. The material is most often presented in the form of beads, e.g. a particulate material having an average diameter of in the range of 0.1-1000 $\mu$m, or in the form of sticks, membranes, pellets, monoliths, etc.

The most common arrangement of the hydrophobic interaction chromatography material is in a column format. Arrangement in a batch container is of course also possible.

The drug substance of the Factor VII polypeptide is typically obtained directly from a preceding purification step, or from a preceding purification step with subsequent adjustment of pH, ionic strength, chelation of divalent metal ions, etc., if desirable or beneficial.

The contacting of the drug substance of the Factor VII polypeptide is typically conducted according to conventional protocols, i.e. the concentration, temperature, ionic strength, etc. of the drug substance may be as usual, and the hydrophobic interaction chromatography material may be washed and equilibrated before application as usual.

The load of Factor VII polypeptide is typically at least 250 mg per litre of matrix (HIC material), such as in the range of 0.5-10.0 g, e.g. 2.0-5.0 g, Factor VII polypeptide per litre of matrix (hydrophobic interaction chromatography material in wet form), and the drug substance is typically loaded at a flow of 1-50 column volumes per hour.

The pH of the drug substance before and upon application to the hydrophobic interaction chromatography material appears to play a relevant role for the formation of Late eluting peaks. Thus, it is preferred that the drug substance is in liquid form and has a pH in the range of 3.0-10.0, such as in the range of 3.0-7.0, or 6.5-10.0, upon application to the hydrophobic interaction chromatography material. In some interesting embodiments, the drug substance has a pH of in the range of 4.0-7.0, or in the range of 7.0-9.0, or in the range of 4.5-8.5. A preferred pH range would be 5.0-6.5.

The content of calcium ions may play a role in connection with the stability of the Factor VII polypeptide. In some preferred embodiments, the drug substance in step (a) has a concentration of calcium ions of at least 5 mM, such as in the range of 5-100 mM. In such instances, a preferred pH range would be 5.0-9.5.

Typically, the conductivity is at least 40 mS/cm, such as at least 50 mS/cm, such as at least 100 mS/cm such at least 200 mS/cm.

The temperature of the drug substance is typically 0-25°C, such as around 2-10°C or 2-8°C.

The temperature of the hydrophobic interaction chromatography material with the bound Factor VII polypeptide is typically 0-25°C, such as around 2-10°C or 2-8°C, e.g. kept within a specified range by using a cooling jacket and solutions of controlled temperature.

*Step (b) - Washing step (optional)*

After binding of the drug substance of the Factor VII polypeptide to the hydrophobic interaction chromatography materials, a washing step (b) is typically conducted in order to remove a substantial fraction of the Late eluting peaks from the hydrophobic interaction chromatography material. By this step, the remaining (bound) fraction of the Factor VII polypeptide on the hydrophobic interaction chromatography material will have a much lower abundance of Late eluting peaks.

The content of a salt and/or zwitterions in the washing buffer has also turned out to provide certain advantages. Thus, preferably, the washing buffer in step (b) comprises a salt and/or a zwitterions in a concentration of in the range of 0.0-0.1 M or in the range of 0.5 M to 85% of the saturation concentration for the respective salt. Most preferably, the concentration of the salt and/or zwitterions in the washing buffer is within $\pm 0.1$ M of the concentration of the salt in the drug substance in step (a). In a particularly preferred embodiment, the washing buffer comprises a salt in a concentration of in the range of 0.5 M to 85% of the saturation concentration, e.g. 0.7-2.2 M.

This washing step (b) is preferably conducted with a washing buffer having a pH in the range of 2.0-6.9. In some interesting embodiments, the washing buffer has a pH in the range of 3.0-10.0, such as in the range of 3.0-7.0, or 6.5-10.0, upon application to the hydrophobic interaction chromatography material. In some interesting embodiments, the washing buffer has a pH of in the range of 4.0-7.0, or in the range of 7.0-9.0, or in the range of 4.5-8.5.

As above in step (a), the presence of calcium ions appears to play a certain role. Thus, the washing buffer in step (b) typically has a concentration of calcium ions of at least 5 mM, such as in the range of 5-100 mM.

The washing step (b) is typically conducted at a flow of 1-50 column volumes per hour.

The washing buffer is typically an aqueous solution comprising a buffering agent, typically a buffering agent comprising at least one component selected from the groups consisting of acids and salts of MES, PIPES, ACES, BES, TES, HEPES, TRIS, histidine, imidazole, glycine, glycylglycine, glycinamide, phosphoric acid, acetic acid (e.g. sodium acetate), lactic acid, glutaric acid, citric acid, tartaric acid, malic acid, maleic acid, and succinic acid. It should be understood that the buffering agent may comprise a mixture of two or more components, wherein the mixture is able to provide a pH value in the specified range. As examples can be mentioned acetic acid and sodium acetate, etc.

It should be understood that the washing step (b) may be conducted by using one, two or several different washing buffers, or by the application of a gradient washing buffer.

It should also be noted that the washing step and the elution step need not to be discrete steps, but may be combined, in particular if a gradient elution buffer is utilised in the elution step.

*Step (c) -Elution step*

After the washing step(s) (c), the hydrophobic interaction chromatography material is eluted with an elution buffer, and a purified drug substance of the Factor VII polypeptide is collected as an eluate.

A great deal of variability is possible for the elution step (c). As for the before-mentioned steps, the elution buffer preferably also comprises a salt and/or a zwitterion.

The type of elution is not particularly critical, thus, it is, e.g., possible to elute with a elution buffer comprising a stepwise decreasing gradient of the salt and/or zwitterion, elute with a linear decreasing gradient of the salt (or a gradient-hold-gradient profile, or other variants), or to use a pH gradient, or to us a temperature gradient, or a combination of the before-mentioned. Alternatively, a gradient of a calcium chelating compound (e.g. EDTA, citrate, malonate, etc.) or a solvent less polar than water (e.g. aqueous solutions comprising ethanol, PEG, 2-propanol, or the like), may be used as the elution buffer.

In one embodiment, the elution buffer comprises a salt in an initial concentration of in the range of 0.7-2.2 M.

In one embodiment, the elution buffer does not contain a calcium-chelating compound. In another embodiment, the elution buffer does not contain EDTA and/or citrate. In one embodiment, the elution buffer contains a calcium salt; in another embodiment is does not contain a calcium salt. In one embodiment, the method according to the invention is carried out in the presence of a calcium salt; in another embodiment, the method according to the invention is carried out in the absence of a calcium salt.

Hence, in a currently most preferred embodiment, the elution buffer in step (c) is a gradient buffer with respect to the an ammonium salt, wherein the initial concentration of the ammonium salt of the gradient buffer is in the range of 1.7-2.2 M, and the final concentration of the ammonium salt of the gradient buffer is in the range of 0.0-1.6 M.

The conductivity of the final elution buffer is preferably lower than the conductivity of the composition comprising the drug substance in step (a).

In most instances, the elution buffer in step (c) typically has a pH as in step (a) and (b).

Also preferred are the embodiments where the elution buffer in step (c) has a concentration of calcium ions of at least 5 mM, such as in the range of 5-100 mM.

The elution step (c) is typically conducted at a flow of 1-50 column volumes per hour.

The term "purified drug substance" means that the resulting drug substance, i.e. the drug substance collected in step (c), has a lower content of product-related impurities, such as Late eluting peaks, than the drug substance applied in step (a). The term "purification" refers to the process wherein a purified drug substance can be obtained, i.e. the process of the present invention.

Typically, the process of the present invention is capable of reducing the content of product-related impurities, including Late eluting peaks, with at least 50% compared to - or relative to - the initial content of said impurities in the drug substance, however more preferably, and also realistically, the relative reduction is at least 60%, such as at least 70% or even at least 80% or at least 85%.

In one embodiment, the product-related impurities are Late eluting peaks. In another embodiment, the product-related impurities are Factor VII polypeptides lacking one or more N-linked glycan(s). In other embodiments, the product-related impurities are oxidized forms, proteolytically degraded forms (auto-catalyzed degradation of the heavy chain of the molecule) or aggregates having a higher molecular weight than the Factor VII polypeptide of interest (including dimers, oligomers, polymers). In another embodiment the product-related impurities contains a mixture of one or more of the above-mentioned forms, peaks and glycan-lacking forms.

Furthermore, it has been found that the process of the invention very efficiently renders it possible to remove product-related impurities, such as Late eluting peaks from drug substances of Factor VII polypeptides, and also renders it possible to suppress the formation of such Late eluting peaks.

Thus, in preferred embodiments, the purified drug substance of the Factor VII polypeptide collected in step (c) comprises less than 50% of Late eluting peaks compared to the drug substance in step (a), e.g. less than 40%, or less than 30%.

In one embodiment, the content of oxidized forms in the purified drug substance of the Factor VII polypeptide collected in step (c) is reduced with at least 35%, such as e.g. at least 40 or 45%, compared to the content of the drug substance in step (a). In another embodiment, the content of proteolytically degraded forms in the purified drug substance of the Factor VII polypeptide collected in step (c) is reduced with at least 30%, such as e.g. at least 32% or 38%, compared to the content in the drug substance in step (a). In another embodiment, the content of Factor VII polypeptides lacking one or more N-linked glycans (des-N-glycan forms) in the purified drug substance of the Factor VII polypeptide collected in step (c) is reduced with at least 40%, such as e.g. at least 50%, 60%, 70% or 75%, compared to the content in the drug substance in step (a).

More particularly, the purified drug substance of the Factor VII polypeptide comprises at the most 5%, such as at the most 2.0%, or at the most 1.5%, preferably, at the most 1.0% or at the most 5%, of Late eluting peaks.

Usually, the hydrophobic interaction chromatography material is regenerated for the purpose of subsequent use by a sequence of steps.

It should be noted that the washing step and the elution step need not to be discrete steps, but may be combined, in particular if a gradient elution buffer is utilised in the elution step.

*Preferred embodiments*

The present process is particularly useful for obtaining a purified drug substance of a Factor VII polypeptide, and if the conditions for the steps (a)-(c) with respect to pH are selected properly, it is even possible to reduce the formation of Late eluting peaks and thereby increase the overall yield of the process.

Thus, a preferred embodiment provides a process for the purification of a drug substance of a Factor VII polypeptide, said drug substance comprising at least 3% of Late eluting peaks, said process comprising the steps of:

(a) contacting the drug substance with a hydrophobic interaction chromatography material under conditions which facilitate binding of a portion of said drug substance to said hydrophobic interaction chromatography material, said drug substance comprising an ammonium salt in a concentration of in the range of 1.5-2.5 M;

(b) washing said hydrophobic interaction chromatography material with a washing buffer comprising the ammonium salt in a concentration of in the range of 1.5-2.5 M, typically substantially the same concentration as in step (a); and

(c) eluting said hydrophobic interaction chromatography material with an elution buffer comprising an ammonium salt, said elution buffer being a gradient buffer with respect to the ammonium salt, and collecting a purified drug substance of the Factor VII polypeptide as an eluate,

wherein steps (b) and (c) may be combined.

Preferably, the elution buffer in step (c) is a gradient buffer with respect to the an ammonium salt, wherein the initial concentration of the ammonium salt of the gradient buffer is in the range of 1.8-2.2 M, and the final concentration of the ammonium salt of the gradient buffer is in the range of 0.0-0.2 M. The ammonium salt is preferably ammonium acetate or ammonium sulphate (when calcium is absent).

*Factor VII polypeptide*

As used herein, the term "Factor VII polypeptide" encompasses wild-type Factor VII (i.e. a polypeptide having the amino acid sequence disclosed in U.S. Patent No. 4,784,950), variants thereof as well as Factor VII-related polypeptides, Factor VII derivatives and Factor VII conjugates. This includes FVII variants, Factor VII-related polypeptides, Factor VII derivatives and Factor VII conjugates exhibiting substantially the same or improved biological activity relative to wild-type human Factor VIIa.

The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. Such variants of Factor VII may exhibit different properties relative to human Factor VII, including stability, phospholipid binding, altered specific activity, and the like. "Factor VII" or "Factor VIIa" within the above definition also includes natural allelic variations that may exist and occur from one individual to another. Also, degree and location of glycosylation or other post-translation modifications may vary depending on the chosen host cells and the nature of the host cellular environment.

The term "Factor VII polypeptide" also encompasses polypeptides, including variants, in which the Factor VIIa biological activity has been substantially modified or somewhat reduced relative to the activity of wild-type Factor VIIa. These polypeptides include, without limitation, Factor VII or Factor VIIa into which specific amino acid sequence alterations have been introduced that modify or disrupt the bioactivity of the polypeptide.

The biological activity of Factor VIIa in blood clotting derives from its ability to (i) bind to Tissue Factor (TF) and (ii) catalyze the proteolytic cleavage of Factor IX or Factor X to produce activated Factor IX or X (Factor IXa or Xa, respectively).

For the purposes of the invention, biological activity of Factor VII polypeptides ("Factor VII biological activity") may be quantified by measuring the ability of a preparation to promote blood clotting, cf. Assay 4 described herein. In this assay, biological activity is expressed as the reduction in clotting time relative to a control sample and is converted to "Factor VII units" by comparison with a pooled human serum standard containing 1 unit/mL Factor VII activity. Alternatively, Factor VIIa biological activity may be quantified by (i) measuring the ability of Factor VIIa or a Factor VII-related polypeptide to produce activated Factor X (Factor Xa) in a system comprising TF embedded in a lipid membrane and Factor X. (Persson et al., J. Biol. Chem. 272:19919-19924, 1997); (ii) measuring Factor X hydrolysis in an aqueous system ("In Vitro Proteolysis Assay", see Assay 2 below); (iii) measuring the physical binding of Factor VIIa or a Factor VII-related polypeptide to TF using an instrument based on surface plasmon resonance (Persson, FEBS Letts. 413:359-363, 1997); (iv) measuring hydrolysis of a synthetic substrate by Factor VIIa and/or a Factor VII-related polypeptide ("In Vitro Hydrolysis Assay", see Assay 1 below); or (v) measuring generation of thrombin in a TF-independent in vitro system (see Assay 3 below).

Factor VII variants having substantially the same or improved biological activity relative to wild-type Factor VIIa encompass those that exhibit at least about 25%, preferably at least about 50%, more preferably at least about 75% and most preferably at least about 90% of the specific activity of Factor VIIa that has been produced in the same cell type, when tested in one or more of a clotting assay (Assay 4), proteolysis assay (Assay 2), or TF binding assay as described above. Factor VII variants having substantially reduced biological activity relative to wild-type Factor VIIa are those that exhibit less than about 25%, preferably less than about 10%, more preferably less than about 5% and most preferably less than about 1% of the specific activity of wild-type Factor VIIa that has been produced in the same cell type when tested in one or more of a clotting assay (Assay 4), proteolysis assay (Assay 2), or TF binding assay as described above. Factor VII variants having a substantially modified biological activity relative to wild-type Factor VII include, without limitation, Factor VII variants that exhibit TF-independent Factor X proteolytic activity and those that bind TF but do not cleave Factor X.

Variants of Factor VII, whether exhibiting substantially the same or better bioactivity than wild-type Factor VII, or, alternatively, exhibiting substantially modified or reduced bioactivity relative to wild-type Factor VII, include, without limitation, polypeptides having an amino acid sequence that differs from the sequence of wild-type Factor VII by insertion, deletion, or substitution of one or more amino acids.

Non-limiting examples of Factor VII variants having substantially the same biological activity as wild-type Factor VII include S52A-FVIIa, S60A-FVIIa (Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVII variants as disclosed in PCT/DK02/00189; and FVII variants exhibiting increased proteolytic stability as disclosed in WO 02/38162 (Scripps Research Institute); FVII variants having a modified Gla-domain and exhibiting an enhanced membrane binding as disclosed in WO 99/20767 (University of Minnesota); and FVII variants as disclosed in WO 01/58935 (Maxygen ApS).

Non-limiting examples of Factor VII variants having increased biological activity compared to wild-type FVIIa include FVII variants as disclosed in WO 01/83725, WO 02/22776, WO 02/077218, WO 03/27147, WO 03/37932; WO 02/38162 (Scripps Research Institute); and FVIIa variants with enhanced activity as disclosed in JP 2001061479 (Chemo-Sero-Therapeutic Res Inst.).

The term "Factor VII polypeptide" also encompasses polypeptides, including variants, in which the Factor VIIa biological activity has been substantially modified or somewhat reduced relative to the activity of wild-type Factor VIIa, but that typically retain a significant level of amino acid sequence identity to Factor VII, such as at least about 70%, at least about 75%, at least about 80%, at least about 85%, at least about 90%, at least about 93%, at least about 95%, or more (e.g., about 97, 98, or 99% identity). These polypeptides include, without limitation, Factor VII or Factor VIIa into which specific amino acid sequence alterations have been introduced that modify or disrupt the bioactivity of the polypeptide (as exemplified further herein).

The term "Factor VII derivative" as used herein, is intended to designate a FVII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, acylation, ester formation or amide formation or the like. This includes but is not limited to PEGylated human Factor VIIa, cysteine-PEGylated human Factor VIIa and variants thereof. Non-limiting examples of Factor VII derivatives includes GlycoPegylated FVII derivatives as disclosed in WO 03/31464 and US Patent applications US 20040043446, US 20040063911, US 20040142856, US 20040137557, and US 20040132640 (Neose Technologies, Inc.); FVII conjugates as disclosed in WO 01/04287, US patent application 20030165996, WO 01/58935, WO 03/93465 (Maxygen ApS) and WO 02/02764, US patent application 20030211094 (University of Minnesota); and FVII variants as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

The term "increased-" or "improved biological activity" refers to FVII polypeptides with i) substantially the same or increased proteolytic activity compared to recombinant wild type human Factor VIIa or ii) to FVII polypeptides with substantially the same or increased TF binding activity compared to recombinant wild type human Factor VIIa or iii) to FVII polypeptides with substantially the same or increased half life in blood plasma compared to recombinant wild type human Factor VIIa. The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

Ngn-limiting examples of Factor VII variants having substantially reduced or modified biological activity relative to wild-type Factor VII include R152E-FVIIa (Wildgoose et al., Biochem 29:3413-3420, 1990), S344A-FVIIa (Kazama et al., J. Biol. Chem. 270:66-72, 1995), FFR-FVIIa (Holst et al., Eur. J. Vasc. Endovasc. Surg. 15:515-520, 1998), and Factor VIIa lacking the Gla domain, (Nicolaisen et al., FEBS Letts. 317:245-249, 1993).

Examples of Factor VII polypeptides include, without limitation, wild-type Factor VII, L305V-FVII, L305V/M306D/D309S-FVII, L305I-FVII, L305T-FVII, F374P-FVII, V158T/M298Q-FVII, V158D/E296V/M298Q-FVII, K337A-FVII, M298Q-FVII, V158D/M298Q-FVII, L305V/K337A-FVII, V158D/E296V/M298Q/L305V-FVII, V158D/E296V/M298Q/K337A-FVII, V158D/E296V/M298Q/L305V/K337A-FVII, K157A-FVII, E296V-FVII, E296V/M298Q-FVII, V158D/E296V-FVII, V158D/M298K-FVII, and S336G-FVII, L305V/K337A-FVII, L305V/V158D-FVII, L305V/E296V-FVII, L305V/M298Q-FVII, L305V/V158T-FVII, L305V/K337A/V158T-FVII, L305V/K337A/M298Q-FVII, L305V/K337A/E296V-FVII, L305V/K337A/V158D-FVII, L305V/V158D/M298Q-FVII, L305V/V158D/E296V-FVII, L305V/V158T/M298Q-FVII, L305V/V158T/E296V-FVII, L305V/E296V/M298Q-FVII, L305V/V158D/E296V/M298Q-FVII, L305V/V158T/E296V/M298Q-FVII, L305V/V158T/K337A/M298Q-FVII, L305V/V158T/E296V/K337A-FVII, L305V/V158D/K337A/M298Q-FVII, L305V/V158D/E296V/K337A-FVII, L305V/V158D/E296V/M298Q/K337A-FVII, L305V/V158T/E296V/M298Q/K337A-FVII, S314E/K316H-FVII, S314E/K316Q-FVII, S314E/L305V-FVII, S314E/K337A-FVII, S314E/V158D-FVII, S314E/E296V-FVII, S314E/M298Q-FVII, S314E/V158T-FVII, K316H/L305V-FVII, K316H/K337A-FVII, K316H/V158D-FVII, K316H/E296V-FVII, K316H/M298Q-FVII, K316H/V158T-FVII, K316Q/L305V-FVII, K316Q/K337A-FVII, K316Q/V158D-FVII, K316Q/E296V-FVII, K316Q/M298Q-FVII, K316Q/V158T-FVII, S314E/L305V/K337A-FVII, S314E/L305V/V158D-FVII, S314E/L305V/E296V-FVII, S314E/L305V/M298Q-FVII, S314E/L305V/V158T-FVII, S314E/L305V/K337A/V158T-FVII, S314E/L305V/K337A/M298Q-FVII, S314E/L305V/K337A/E296V-FVII, S314E/L305V/K337A/V158D-FVII, S314E/L305V/V158D/M298Q-FVII, S314E/L305V/V158D/E296V-FVII, S314E/L305V/V158T/M298Q-FVII, S314E/L305V/V158T/E296V-FVII, S314E/L305V/E296V/M298Q-FVII, S314E/L305V/V158D/E296V/M298Q-FVII, S314E/L305V/V158T/E296V/M298Q-FVII, S314E/L305V/V158T/K337A/M298Q-FVII, S314E/L305V/V158T/E296V/K337A-FVII, S314E/L305V/V158D/K337A/M298Q-FVII, S314E/L305V/V158D/E296V/K337A-FVII, S314E/L305V/V158D/E296V/M298Q/K337A-FVII, S314E/L305V/V158T/E296V/M298Q/K337A-FVII, K316H/L305V/K337A-FVII, K316H/L305V/V158D-FVII, K316H/L305V/E296V-FVII, K316H/L305V/M298Q-FVII, K316H/L305V/V158T-FVII, K316H/L305V/K337A/V158T-FVII, K316H/L305V/K337A/M298Q-FVII, K316H/L305V/K337A/E296V-FVII, K316H/L305V/K337A/V158D-FVII, K316H/L305V/V158D/M298Q-FVII, K316H/L305V/V158D/E296V-FVII, K316H/L305V/V158T/M298Q-FVII, K316H/L305V/V158T/E296V-FVII, K316H/L305V/E296V/M298Q-FVII, K316H/L305V/V158D/E296V/M298Q-FVII, K316H/L305V/V158T/E296V/M298Q-FVII, K316H/L305V/V158T/K337A/M298Q-FVII, K316H/L305V/V158T/E296V/K337A-FVII, K316H/L305V/V158D/K337A/M298Q-FVII, K316H/L305V/V158D/E296V/K337A-FVII, K316H/L305V/V158D/E296V/M298Q/K337A-FVII, K316H/L305V/V158T/E296V/M298Q/K337A-FVII, K316Q/L305V/K337A-FVII, K316Q/L305V/V158D-FVII, K316Q/L305V/E296V-FVII, K316Q/L305V/M298Q-FVII, K316Q/L305V/V158T-FVII, K316Q/L305V/K337A/V158T-FVII, K316Q/L305V/K337A/M298Q-FVII, K316Q/L305V/K337A/E296V-FVII, K316Q/L305V/K337A/V158D-FVII, K316Q/L305V/V158D/M298Q-FVII, K316Q/L305V/V158D/E296V-FVII, K316Q/L305V/V158T/M298Q-FVII, K316Q/L305V/V158T/E296V-FVII, K316Q/L305V/E296V/M298Q-FVII, K316Q/L305V/V158D/E296V/M298Q-FVII, K316Q/L305V/V158T/E296V/M298Q-FVII, K316Q/L305V/V158T/K337A/M298Q-FVII, K316Q/L305V/V158T/E296V/K337A-FVII, K316Q/L305V/V158D/K337A/M298Q-FVII, K316Q/L305V/V158D/E296V/K337A-FVII, K316Q/L305V/V158D/E296V/M298Q/K337A-FVII, K316Q/L305V/V158T/E296V/M298Q/K337A-FVII, F374Y/K337A-FVII, F374Y/V158D-FVII, F374Y/E296V-FVII, F374Y/M298Q-FVII, F374Y/V158T-FVII, F374Y/S314E-FVII, F374Y/L305V-FVII, F374Y/L305V/K337A-FVII, F374Y/L305V/V158D-FVII, F374Y/L305V/E296V-FVII, F374Y/L305V/M298Q-FVII, F374Y/L305V/V158T-FVII, F374Y/L305V/S314E-FVII, F374Y/K337A/S314E-FVII, F374Y/K337A/V158T-FVII, F374Y/K337A/M298Q-FVII, F374Y/K337A/E296V-FVII, F374Y/K337A/V158D-FVII, F374Y/V158D/S314E-FVII, F374Y/V158D/M298Q-FVII, F374Y/V158D/E296V-FVII, F374Y/V158T/S314E-FVII, F374Y/V158T/M298Q-FVII, F374Y/V158T/E296V-FVII, F374Y/E296V/S314E-FVII, F374Y/S314E/M298Q-FVII, F374Y/E296V/M298Q-FVII, F374Y/L305V/K337A/V158D-FVII, F374Y/L305V/K337A/E296V-FVII, F374Y/L305V/K337A/M298Q-FVII, F374Y/L305V/K337A/V158T-FVII, F374Y/L305V/K337A/S314E-FVII, F374Y/L305V/V158D/E296V-FVII, F374Y/L305V/V158D/M298Q-FVII, F374Y/L305V/V158D/S314E-FVII, F374Y/L305V/E296V/M298Q-FVII, F374Y/L305V/E296V/V158T-FVII, F374Y/L305V/E296V/S314E-FVII, F374Y/L305V/M298Q/V158T-FVII, F374Y/L305V/M298Q/S314E-FVII, F374Y/L305V/V158T/S314E-FVII, F374Y/K337A/S314E/V158T-FVII, F374Y/K337A/S314E/M298Q-FVII, F374Y/K337A/S314E/E296V-FVII, F374Y/K337A/S314E/V158D-FVII, F374Y/K337A/V158T/M298Q-FVII, F374Y/K337A/V158T/E296V-FVII, F374Y/K337A/M298Q/E296V-FVII, F374Y/K337A/M298Q/V158D-FVII, F374Y/K337A/E296V/V158D-FVII, F374Y/V158D/S314E/M298Q-FVII, F374Y/V158D/S314E/E296V-FVII, F374Y/V158D/M298Q/E296V-FVII, F374Y/V158T/S314E/E296V-FVII, F374Y/V158T/S314E/M298Q-FVII, F374Y/V158T/M298Q/E296V-FVII, F374Y/E296V/S314E/M298Q-FVII, F374Y/L305V/M298Q/K337A/S314E-FVII, F374Y/L305V/E296V/K337A/S314E-FVII, F374Y/E296V/M298Q/K337A/S314E-FVII, F374Y/L305V/E296V/M298Q/K337A-FVII, F374Y/L305V/E296V/M298Q/S314E-FVII, F374Y/V158D/E296V/M298Q/K337A-FVII, F374Y/V158D/E296V/M298Q/S314E-FVII, F374Y/L305V/V158D/K337A/S314E-FVII, F374Y/V158D/M298Q/K337A/S314E-FVII, F374Y/V158D/E296V/K337A/S314E-FVII, F374Y/L305V/V158D/E296V/M298Q-FVII, F374Y/L305V/V158D/M298Q/K337A-FVII, F374Y/L305V/V158D/E296V/K337A-FVII, F374Y/L305V/V158D/M298Q/S314E-FVII, F374Y/L305V/V158D/E296V/S314E-FVII, F374Y/V158T/E296V/M298Q/K337A-FVII, F374Y/V158T/E296V/M298Q/S314E-FVII, F374Y/L305V/V158T/K337A/S314E-FVII, F374Y/V158T/M298Q/K337A/S314E-FVII, F374Y/V158T/E296V/K337A/S314E-FVII, F374Y/L305V/V158T/E296V/M298Q-FVII, F374Y/L305V/V158T/M298Q/K337A-FVII, F374Y/L305V/V158T/E296V/K337A-FVII, F374Y/L305V/V158T/M298Q/S314E-FVII, F374Y/L305V/V158T/E296V/S314E-FVII, F374Y/E296V/M298Q/K337A/V158T/S314E-FVII, F374Y/V158D/E296V/M298Q/K337A/S314E-FVII, F374Y/L305V/V158D/E296V/M298Q/S314E-FVII, F374Y/L305V/E296V/M298Q/V158T/S314E-FVII, F374Y/L305V/E296V/M298Q/K337A/V158T-FVII, F374Y/L305V/E296V/K337A/V158T/S314E-FVII, F374Y/L305V/M298Q/K337A/V158T/S314E-FVII, F374Y/L305V/V158D/E296V/M298Q/K337A-FVII, F374Y/L305V/V158D/E296V/K337A/S314E-FVII, F374Y/L305V/V158D/M298Q/K337A/S314E-FVII, F374Y/L305V/E296V/M298Q/K337A/V158T/S314E-FVII, F374Y/L305V/V158D/E296V/M298Q/K337A/S314E-FVII, S52A-Factor VII, S60A-Factor VII; R152E-Factor VII, S344A-Factor VII, Factor VIIa lacking the Gla domain; and P11Q/K33E-FVII, T106N-FVII, K143N/N145T-FVII, V253N-FVII, R290N/A292T-FVII, G291N-FVII, R315N/V317T-FVII, K143N/N145T/R315N/V317T-FVII; and FVII having substitutions, additions or deletions in the amino acid sequence from 233Thr to 240Asn, FVII having substitutions, additions or deletions in the amino acid sequence from 304Arg to 329Cys, and FVII having substitutions, deletions, or additions in the amino acid sequence Ile153-Arg223.

Thus, substitution variants in a factor VII polypeptide include, without limitation substitutions in positions P10, K32, L305, M306, D309, L305, L305, F374, V158, M298, V158, E296, K337, M298, M298, S336, S314, K316, K316, F374, S52, S60, R152, S344, T106, K143, N145, V253, R290, A292, G291, R315, V317, and substitutions, additions or deletions in the amino acid sequence from T233 to N240 or from R304 to C329; or from I153 to R223, or combinations thereof, in particular variants such as P10Q, K32E, L305V, M306D, D309S, L305I, L305T, F374P, V158T, M298Q, V158D, E296V, K337A, M298Q, M298K, S336G, S314E, K316H, K316Q, F374Y, S52A, S60A, R152E, S344A, T106N, K143N, N145T, V253N, R290N, A292T, G291N, R315N, V317T, and substitutions, additions or deletions in the amino acid sequence from T233 to N240, or from R304 to C329, or from I153 to R223, or combinations thereof.

In some embodiments, the Factor VII polypeptide is recombinantly made human Factor VIIa (rhVIIa).

In other embodiments, the Factor VII polypeptide is a Factor VII sequence variant.

In some embodiments, the Factor VII polypeptide has a glycosylation different from wild-type human Factor VII.

In various embodiments, e.g. those where the Factor VII polypeptide is a Factor VII-related polypeptide or a Factor VII sequence variant, the ratio between the activity of the Factor VII polypeptide and the activity of native human Factor VIIa (wild-type FVIIa) is at least about 1.25, preferably at least about 2.0, or 4.0, most preferred at least about 8.0, when tested in the "In Vitro Proteolysis Assay" (Assay 2) as described in the present specification.

In some embodiments, the Factor VII polypeptides are Factor VII-related polypeptides, in particular variants, wherein the ratio between the activity of said Factor VII polypeptide and the activity of native human Factor VIIa (wild-type FVIIa) is at least about 1.25 when tested in the "In Vitro Hydrolysis Assay" (see Assay 1 below); in other embodiments, the ratio is at least about 2.0; in further embodiments, the ratio is at least about 4.0.

*Use of the purified drug substance of the Factor VII polypeptide*

After collection of the fractions corresponding to the purified drug substance of the Factor VII polypeptide, may be formulated into a solution, which may be dispensed into vials and freeze-dried or store as such. As an illustrative example of a final product corresponding to the commercially available, recombinantly-made FVII polypeptide composition NovoSeven® (Novo Nordisk A/S, Denmark), can be mentioned a vial (1.2 mg) containing 1.2 mg recombinant human Factor VIIa, 5.84 mg NaCl, 2.94 mg CaCl$_2$, 2 H$_2$O, 2.64 mg GlyGly, 0.14 mg polysorbate 80, and 60.0 mg mannitol. This product is reconstituted to pH 5.5 by 2.0 mL water for injection (WFI) prior to use. When reconstituted, the protein solution is stable for use for 24 hours.

The overall manufacture of recombinant activated Factor VII (rFVIIa) is described by Jurlander, et al. in Seminars in Thrombosis and Hemostasis, Vol. 27, No. 4, 2001.

EXPERIMENTALS

*General methods*

Assays suitable for determining biological activity of Factor VII polypeptides

Factor VII polypeptides useful in accordance with the present invention may be selected by suitable assays that can be performed as simple preliminary in vitro tests. Thus, the present specification discloses a simple test (entitled "In Vitro Hydrolysis Assay") for the activity of Factor VII polypeptides.

1st generation clot assay

The activity of the Factor VII polypeptides may be measured using a one-stage clot assay essentially as described in WO 92/15686 or US 5,997,864. Briefly, the sample to be tested is diluted in 50 mM Tris (pH 7.5), 0.1% BSA and 100 μL is incubated with 100 μL of Factor VII deficient plasma and 200 μL of thromboplastin C containing 10 mM Ca2+. Clotting times are measured and compared to a standard curve using a reference standard or a pool of citrated normal human plasma in serial dilution.

<u>In Vitro Hydrolysis Assay (Assay 1)</u>

Native (wild-type) Factor VIIa and Factor VII polypeptide (both hereinafter referred to as "Factor VIIa") may be assayed for specific activities. They may also be assayed in parallel to directly compare their specific activities. The assay is carried out in a microtiter plate (MaxiSorp, Nunc, Denmark). The chromogenic substrate D-Ile-Pro-Arg-p-nitroanilide (S-2288, Chromogenix, Sweden), final concentration 1 mM, is added to Factor VIIa (final concentration 100 nM) in 50 mM HEPES, pH 7.4, containing 0.1 M NaCl, 5 mM $CaCl_2$ and 1 mg/mL bovine serum albumin. The absorbance at 405 nm is measured continuously in a SpectraMax™ 340 plate reader (Molecular Devices, USA). The absorbance developed during a 20-minute incubation, after subtraction of the absorbance in a blank well containing no enzyme, is used for calculating the ratio between the activities of Factor VII polypeptide and wild-type Factor VIIa:

$$Ratio = (A405 \text{ nm Factor VII polypeptide})/(A405 \text{ nm Factor VIIa wild-type}).$$

Based thereon, Factor VII polypeptides with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

The activity of the Factor VII polypeptides may also be measured using a physiological substrate such as Factor X ("In Vitro Proteolysis Assay"), suitably at a concentration of 100-1000 nM, where the Factor Xa generated is measured after the addition of a suitable chromogenic substrate (eg. S-2765). In addition, the activity assay may be run at physiological temperature.

<u>In Vitro Proteolysis Assay (Assay 2)</u>

Native (wild-type) Factor VIIa and Factor VII polypeptide (both hereinafter referred to as "Factor VIIa") are assayed in parallel to directly compare their specific activities. The assay is carried out in a microtiter plate (MaxiSorp, Nunc, Denmark). Factor VIIa (10 nM) and Factor X (0.8 microM) in 100 μL 50 mM HEPES, pH 7.4, containing 0.1 M NaCl, 5 mM $CaCl_2$ and 1 mg/mL bovine serum albumin, are incubated for 15 min. Factor,X cleavage is then stopped by the addition of 50 μL 50 mM HEPES, pH 7.4, containing 0.1 M NaCl, 20 mM EDTA and 1 mg/mL bovine serum albumin. The amount of Factor Xa generated is measured by the addition of the chromogenic substrate Z-D-Arg-Gly-Arg-p-nitroanilide (S-2765, Chromogenix, Sweden), final concentration 0.5 mM. The absorbance at 405 nm is measured continuously in a SpectraMax™ 340 plate reader (Molecular Devices, USA). The absorbance developed during 10 minutes, after subtraction of the absorbance in a blank well containing no FVIIa, is used for calculating the ratio between the proteolytic activities of Factor VII polypeptide and wild-type Factor VIIa:

$$Ratio = (A405 \text{ nm Factor VII polypeptide})/(A405 \text{ nm Factor VIIa wild-type}).$$

Based thereon, a Factor VII polypeptide with an activity lower than, comparable to, or higher than native Factor VIIa may be identified, such as, for example, Factor VII polypeptides where the ratio between the activity of the Factor VII polypeptide and the activity of native Factor VII (wild-type FVII) is about 1.0 versus above 1.0.

<u>Thrombin generation assay (Assay 3)</u>

The ability of a Factor VII polypeptides to generate thrombin can be measured in an assay (Assay 3) comprising all relevant coagulation Factors and inhibitors at physiological concentrations (minus Factor VIII when mimicking hemophilia A conditions) and activated platelets (as described on p. 543 in Monroe et al. (1997) Brit. J. Haematol. 99, 542-547 which is hereby incorporated as reference).

<u>One-stage Coagulation Assay (Clot Assay) (Assay 4)</u>

Factor VII polypeptides may also be assayed for specific activities ("clot activity") by using a one-stage coagulation assay (Assay 4). For this purpose, the sample to be tested is diluted in 50 mM PIPES-buffer (pH 7.2), 1% BSA and 40 μl is incubated with 40 μl of Factor VII deficient plasma and 80 μl of human recombinant tissue factor containing 10 mM $Ca^{2+}$ and synthetic phospholipids. Coagulation times (clotting times) are measured and compared to a standard curve using a reference standard in a parallel line assay.

<u>RP-HPLC assay suitable for determination of "relative retention time" and content of oxidized FVII, heavy chain degraded FVII and "Late eluting peaks"</u>

"Late eluted peaks" are those eluting with a higher retention relative to that of the Factor VII polypeptide of interest in a RP-HPLC assay, e.g. as described in the following:

Reverse phase HPLC was run on an in-house produced butyl-bonded silica column (4.5x250 mm) with a particle size of 5 μm and pore size 300 Å; (comparable results can be obtained with an ACE C4, 5 μm, 300 Å, 4.6x250mm column).
Column temperature: 70°C. A-buffer: 0.1% v/v trifluoracetic acid. B-buffer: 0.09% v/v trifluoracetic acid, 80% v/v acetonitrile.
The column was eluted with a linear gradient from X to (X+13)% B in 30 minutes. X was adjusted so that FVIIa elutes with a retention time of approximately 26 minutes.
Flow rate: 1.0 mL/min. Detection: 214 nm. Load: 25 μg FVIIa.

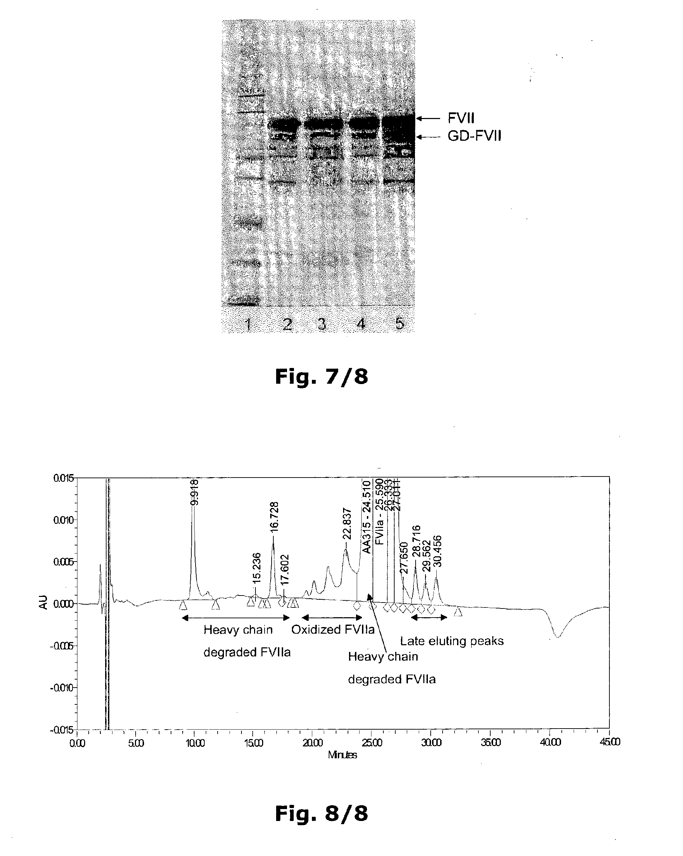

Figure 8 shows a HPLC chromatogram of FVII and its product-related impurities.

<u>Content of aggregates</u>

The content of aggregates is determined by non-denaturing size exclusion HPLC (SEHPLC). Non-denaturing size exclusion chromatography was run on a Waters Protein Pak 300 SW column, 7.5x300 mm using 0.2 M ammonium sulphate, 5% 2-propanol pH 7.0 as mobile phase. Flow rate: 0.5 mL/min. Detection: 215 nm. Load: 25 μg FVIIa.

<u>Determination of content of desGla-Factor VII polypeptide structures</u>

The content of desGla-Factor VII polypeptide structures relative to the full length Factor VII polypeptide structures is determined by SDS-PAGE. 150 μl of sample is added 50 μl of sample buffer (non-reducing, NuPAGE) and boiled for 5 mins. A 10 μl sample is loaded onto a 12% BisTris NuPAGE Gel (Invitrogen). The gel is run at 200 Volts, 120 mA for 55 mins. The gel is stained using coomassie brilliant blue solution, destained and dried. The relative desGla-Factor VII polypeptide content is calculated as the area of the desGla-Factor VII polypeptide band divided by the areas of the Factor VII polypeptide band at approx. 50 kDa and desGla-Factor VII polypeptide band at approx. 45 kDa.

Alternatively, the content of desGla-Factor VII polypeptide structures may be determined by anion exchange HPLC. The method separates Gla-domain-less Factor VII polypeptides from intact Factor VII polypeptides. The content of Gla-domain-less Factor VII polypeptides is expressed in % of the Factor VII polypeptide related peak area. As analytical column is used a DNAPac PA-100, 250x4 mm (Dionex Corp.). The column is eluted with a linear gradient from 0-0.5 M ammonium acetate at pH 9.0 over 30 minutes at a flow of 1.0 mL/min. The absorbance at 280 nm of the effluent is monitored.

<u>Determination of content of Factor VII polypeptide structures lacking one or more N-linked glycans</u>

The content of Factor VII polypeptide structures lacking one or both N-linked glycans may be determined using, e.g., high-performance liquid chromatography (HPLC); capillary electrophoresis (CE); or mass spectrometry (MALDI-MS and LC-MS).

For example, when using MALDI-MS, peaks appearing with mass of about 50 kDa, 47.5 kDa and 45 kDa designate Factor VII polypeptides having both N-linked glycans and Factor VII lacking one or both glycans, respectively.

The pattern of N-linked oligosaccharides may be determined using any method known in the art, including, without limitation: high-performance liquid chromatography (HPLC); capillary electrophoresis (CE); nuclear magnetic resonance (NMR); mass spectrometry (MS) using ionization techniques such as fast-atom bombardment, electrospray, or matrix-assisted laser desorption (MALDI); gas chromatography (GC); and treatment with exoglycosidases in conjunction with anion-exchange (AIE)-HPLC, size-exclusion chromatography (SEC), or MS. See, e.g., Weber et al., Anal. Biochem. 225:135 (1995); Klausen et al., J. Chromatog. 718:195 (1995); Morris et al., in Mass Spectrometry of Biological Materials, McEwen et al., eds., Marcel Dekker, (1990), pp 137-167; Conboy et al., Biol. Mass Spectrom. 21:397, 1992; Hellerqvist, Meth. Enzymol. 193:554 (1990); Sutton et al., Anal. Biohcem. 318:34 (1994); Harvey et al., Organic Mass Spectrometry 29:752 (1994).

Following resolution of Factor VII glycoforms using any of the above methods, the resolved species are assigned to different groups (i)-(iii). The relative content of each of (i)-(iii) is calculated as the sum of the glycoforms assigned to that group relative to the total content of glycoforms in the sample.

Examples

The following examples illustrate the invention. These examples are included for illustrative purposes only and are not intended in any way to limit the scope of the invention claimed.

Example 1 - Reduction of heavy chain degraded and oxidized rhFVII by HIC purification of rhFVIIa at pH 6 using TSK Phenyl 5PW

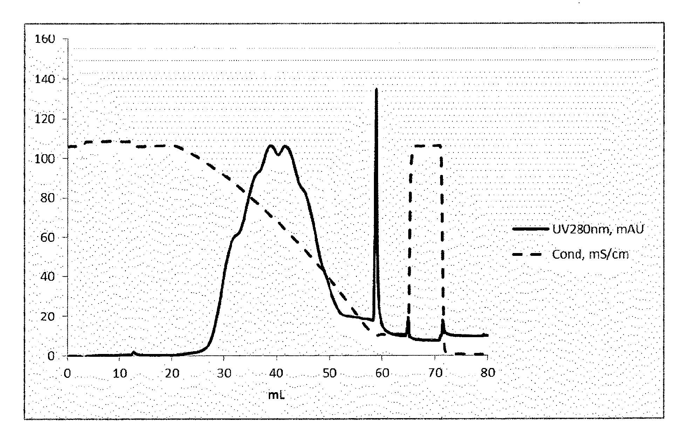

5 mg of highly pure rhFVIIa was added $NH_4$-acetate to a final concentration of 1.8 M and $CaCl_2$ to a final concentration of 10 mM and methionine to a final concentration of 10 mM. pH was adjusted to pH 6.0. This sample was added to a column (0.5 cm in inner diameter x 10.0 cm length = 2 ml column volume (CV)) packed with Toso Haas TSK-Gel phenyl 5 PW, equilibrated with 5 CV 1.8 M $NH_4$-acetate, 10 mM $CaCl_2$, 10 mM methionine, pH 6.0 (load 1.6 g/L). The column was washed with 3 CV 1.8 M $NH_4$-acetate, 10 mM $CaCl_2$, 10 mM methionine, pH 6.0. Elution was performed using a 18 CV linear gradient from 1.8 M $NH_4$-acetate to 50 mM $NH_4$-acetate in a buffer containing 10 mM $CaCl_2$, 10 mM methionine at pH 6.0. Though peak collection at approximately 65% of maximum absorbance (at 280 nm) on the leading edge and at approximately 20% of maximum absorbance on the trailing edge a pool was collected, chromatogram in Figure 1.

The purification was performed at a flow rate between 6 and 12 CV/h and at a temperature of 5°C. The column was regenerated with 50 mM citrate, pH 7.0 and 0.5 M NaOH.

Analysis of the pool and the applied sample by analytical RP-HPLC showed reduction of heavy chain degraded and oxidized rhFVII as shown in Table 1.

Table 1

| Sample | Heavy chain degradation | Oxidized FVII |
|---|---|---|
| Application | 9.9% | 2.9% |
| Pool | 5.9% | 1.5% |

Heavy chain degraded and oxidized rhFVII were primarily reduced because of their relatively lower retention time than unmodified rhFVII and were thus reduced by cutting on the leading edge.

Example 2 - Performing hydrophobic interaction chromatograph

A sample of rhFVII is added $(NH_4)_2SO_4$ to a final concentration 1 M. pH is adjusted to pH 8.6 buffered with 20 mM Tris. This sample is added to a column packed with Toyopearl butyl 650S, equilibrated with 5 CV 1 M $(NH_4)_2SO_4$, 20 mM Tris, pH 8.6. The column is washed with 5 CV 1 M $(NH_4)_2SO_4$, 20 mM Tris, pH 8.6. Elution is performed using a 20 CV linear gradient from 1.0 M $(NH_4)_2SO_4$ to 0 M $(NH_4)_2SO_4$, in a buffer containing 20 mM Tris, pH 8.6. A FVII containing pool is selected through peak collecting. Heavy chain degraded and oxidized rhFVII is reduced due to their lower retention time compared to native rhFVII. The Late eluting peaks 1, 2 and 3 are reduced due to their relatively higher retention time compared to the native rhFVII.

Example 3 - Reduction of Late eluting peaks by HIC purification of FVIIa analogue at pH 6 using TSK Phenyl 5PW

A 4.7 ml column was packed with TSK Phenyl 5PW (20 $\mu$m) resin and equilibrated with 20 CV of a buffer containing: 2.0 M Ammonium acetate, 10 mM $CaCl_2$, 10 mM Histidine, pH 6.0.

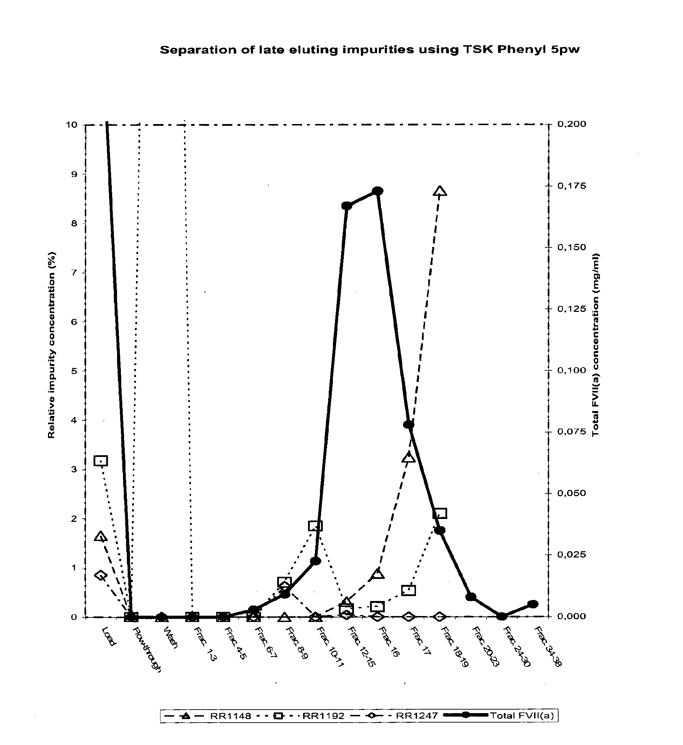

A load consisting of 15 milliliters of 0.25 mg/ml FVIIa analogue in 2.0 M ammonium acetate, 10 mM $CaCl_2$, 10 mM Histidine, pH 6.0 was loaded onto the column, corresponding to a specific load of 0.8 mg/ml resin. The column was washed using 5 CV of the equilibration buffer. The elution was performed using a linear gradient from the wash and equilibration buffer to 10 mM $CaCl_2$, 10 mM Histidine, pH 6.0 over 10 CV followed by a 5 CV hold. 5 ml fractions were collected across the elution peak. The chromatogram is illustrated in Figure 3.

The column was regenerated using 5 CV's of 50 mM tri sodium citrate, pH 7.5 followed by 5 CV's of 1.0 M sodium hydroxide. The flow rate was 6 CV/h throughout the purification and a constant temperature of 5°C was maintained.

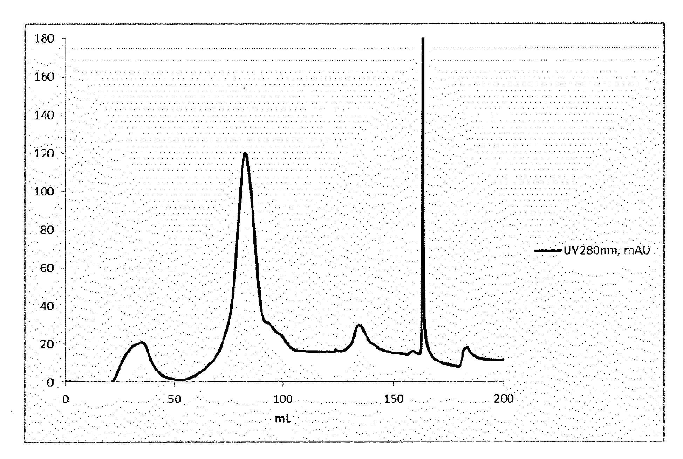

A separation of Late eluting peaks with a relative retention (RR) >1000 was achieved as illustrated in the Figure 2. In total, Late eluting peaks (RR1045, RR1066, RR1119, RR1148, RR1192 and RR1247) were reduced by a factor 2.5. The retention of the peaks refers to the relative retention of the respective peak relative to the main product peak in the analytical RP-HPLC system. The collection of fractions 12-17 resulted in a 62% yield.

Example 4 - The reduction of high-molecular weight compounds

A 2.0 L column was packed with TSK Phenyl 5PW (20 $\mu$m) resin and equilibrated with 20 CV of 2.0 M Ammonium acetate, 10 mM $CaCl_2$, 10 mM Histidine, pH 6.0. 5.3 L of a solution containing 0.751 mg/ml FVIIa analogue in 2.0 M ammonium acetate, 10 mM $CaCl_2$, 10 mM Histidine, pH 6.0 was loaded corresponding to a specific load of 2.0 mg/ml resin. The column was washed using 10 CV of the equilibration buffer. The elution was performed using a linear gradient from the wash and equilibration buffer to 10 mM $CaCl_2$, 10 mM Histidine, pH 6.0 over 10 CV followed by a 6.5 CV hold. A pool fraction was collected from 100 mAu (5 mm light path) on the leading edge to 65 mAu (5 mm light path) on the trailing edge. The pool volume was 9.0 L

The column was regenerated using 5 CV's of 50 mM tri sodium citrate, pH 7.5 followed by 5 CV's of 1.0 M sodium hydroxide. The flow rate was 6 CV/h throughout the purification and a constant temperature of 5°C was maintained.

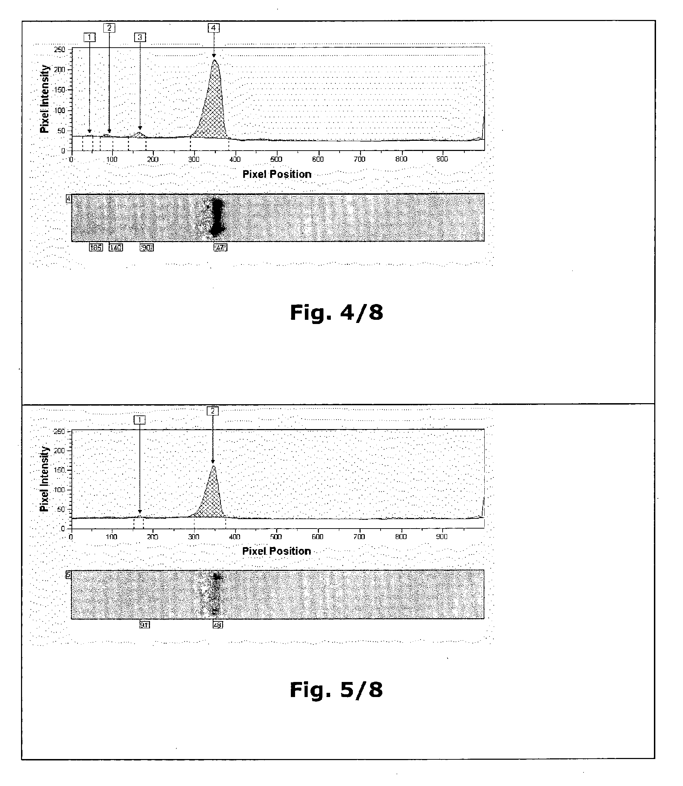

According to SDS-PAGE, the load contained several components with a molecular weight greater than the product. The described purification step reduced all of these to less than half the concentration (see Table 2 and Figures 4 and 5).

Table 2

|  | Mw (kDa) | Load (%) | Pool (%) | Reduction (fold) |
|---|---|---|---|---|
|  | 185 | 0.50 | < 0.50 | **>1.0** |
|  | 148 | 1.11 | < 0.50 | **>2.2** |
|  | 91 | 3.72 | 1.53 | **2.4** |
| **FVIIa analogue** | 48 | 94.67 | 98.47 |  |

Example 5 - Reduction of Late eluting peaks

A sample of the load and pool was analysed by RP-HPLC as described in *General methods,* above. A reduction of both oxidized forms and Late eluting peaks (LE peaks) were seen (see Table 3).

Table 3

|  | HIC Load (%) | HIC pool (%) | Reduction fold |
|---|---|---|---|
| **Sum of LE peaks** | 7.7 | 1.5 | 5.1 |

Example 6 - Reduction of GD-FVII by HIC purification of FVIIa analogue at pH 6 using TSK Phenyl 5PW

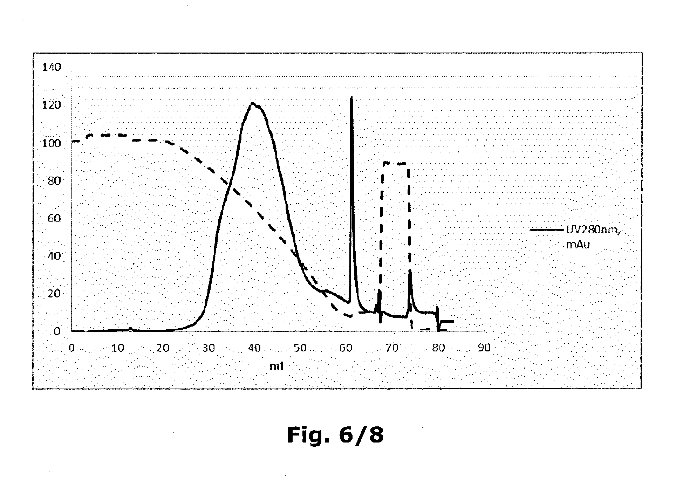

6 mg of recombinant hFVIIa was added $NH_4$-acetate to a final concentration of 1.8 M and $CaCl_2$ to a final concentration of 10 mM. pH was adjusted to pH 6.0. This sample was added to a column (0.5 cm in inner diameter x 10.5 cm length = 2 ml column volume (CV)) packed with Toso Haas TSK-Gel phenyl 5 PW, equilibrated with 5 CV 1.8 M $NH_4$-acetate, 10 mM $CaCl_2$, pH 6.0 (load 1.6 g/L). The column was washed with 3 CV 1.8 M $NH_4$-acetate, 10 mM $CaCl_2$, pH 6.0. Elution was performed using a 18 CV linear gradient from 1.8 M $NH_4$-acetate to 50 mM $NH_4$-acetate in a buffer containing 10 mM $CaCl_2$ at pH 6.0. Three samples were collected; the leading edge, the main peak, end the trailing edge. The main peak was collected through peak collection at approximately 50% of maximum absorbance (at 280 nm) on the leading edge and at approximately 20% of maximum absorbance on the trailing edge. Chromatogram in Figure 6.

The purification was performed at a flow rate of 6-12 CV/h and at a temperature of 5°C. The column was regenerated using 5 CV of 50 mM citrate, pH 7.0 and 5 CV of 0.5 M NaOH.

The relative content of GD-FVII, mw app. 43 kDA, compared to FVII, mw app. 48 kDA, in the three collected samples and in the application sample was analyzed by non-reducing SDS-PAGE, Figure 7, and scanning of the gel, figure. A reduced amount of GD-FVII in the main peak was seen, table 5.

Table 5

| Sample | Relative content of GD-FVII | Lane in figure 7 |
|---|---|---|
| Application | 19% | 2 |
| Leading edge | 18% | 3 |
| Main peak | 15% | 4 |

(continued)

| Sample | Relative content of GD-FVII | Lane in figure 7 |
|---|---|---|
| Tailing edge | 61% | 5 |

Example 7 - Reduction of Late eluting peaks and other product related impurities by HIC purification of FVIIa using TSK Phenyl 5PW at 20 °C

6 mg of recombinant hFVIIa was added NH$_4$-acetate to a final concentration of 1.8 M and CaCl2 to a final concentration of 10 mM. pH was adjusted to pH 6.0. This sample was added to a column (0.5 cm in inner diameter x 10.0 cm length = 2 ml column volume (CV)) packed with Toso Haas TSK-Gel phenyl 5 PW, equilibrated with 5 CV 1.8 M NH$_4$-acetate, 10 mM CaCl$_2$, pH 6.0.

The column was washed with 3 CV 1.8 M NH$_4$-acetate, 10 mM CaCl$_2$, pH 6.0. Elution was performed using a 18 CV linear gradient from 1.8 M NH4-acetate to 50 mM NH4-acetate in a buffer containing 10 mM CaCl2, at pH 6.0.

Though peak collection at approximately 65% of maximum absorbance (at 280 nm) on the leading edge and at approximately 20% of maximum absorbance on the trailing edge. A pool was collected.

The purification was performed at a flow rate between 6 and 12 CV/h and at a temperature of 20°C. The column was regenerated with 50 mM citrate, pH 7.0 and 0.5 M NaOH.

Content of oxidized- and heavy chain degraded FVII and of late eluting peaks was reduced as shown in table 6.

Table 6

| Sample | Oxidized FVII | Heavy chain degraded FVII | Late eluting peaks |
|---|---|---|---|
| Application | 3,9 % | 4,9% | 2,7 % |
| Pool | 2,3 % | 4,5 % | 0,5 % |

**Claims**

1. A process for reducing the content of forms of Factor VII lacking one or more N-linked glycan(s) in a drug substance of a recombinantly made activated Factor VII polypeptide, said process comprising the steps of:

   (a) contacting the drug substance with a hydrophobic interaction chromatography material under conditions which facilitate binding of a portion of said drug substance to said hydrophobic interaction chromatography material, said drug substance comprising a salt selected from the list of: ammonium acetate, ammonium sulphate, ammonium chloride, sodium chloride, sodium acetate, sodium sulphate, potassium acetate, potassium chloride and potassium sulphate, and/or a zwitterions selected from the list of: glycine, alanine, beta-alanine, leucine, and isoleucine, in a concentration of in the range of 0.0-0.1 M or in the range of 0.5 M to 85% of the saturation concentration for the respective salt at the temperature at which step (a) is carried out;
   (b) optionally washing said hydrophobic interaction chromatography material with a washing buffer; and
   (c) eluting said hydrophobic interaction chromatography material with an elution buffer, and collecting a purified drug substance of the activated Factor VII polypeptide as an eluate;

   wherein the content of forms of Factor VII polypeptide lacking one or more N-linked glycans(s) in the purified drug substance collected in step (c), as determined by the methods defined herein, is reduced with at least 50% (w/w) as compared to the drug substance applied in step (a).

2. The process according to claim 1, wherein the hydrophobic interaction chromatography material is selected from the group consisting of resins substituted with butyl ligands and/or phenyl ligands.

3. The process according to any one of the preceding claims, wherein the load of the drug substance in step (a) is in the range of at least 250 mg Factor VII polypeptide per L resin.

4. The process according to any one of the preceding claims, wherein the drug substance in step (a) is in liquid form and has an ionic strength of at least 50 mS/cm.

**5.** The process according to any one of the preceding claims, wherein the drug substance of step (a) comprises a salt in a concentration of in the range of 0.5 M to 85% of the saturation concentration, or 0.7-2.2 M.

**6.** The process according to any one of the preceding claims, wherein the washing buffer in step (b) comprises a salt selected from the list of: ammonium acetate, ammonium sulphate, ammonium chloride, sodium chloride, sodium acetate, sodium sulphate, potassium acetate, potassium chloride and potassium sulphate, and/or a zwitterions selected from the list of: glycine, alanine, beta-alanine, leucine, and isoleucine, in a concentration of in the range of 0.0-0.1 M or in the range of 0.5 M to 85% of the saturation concentration for the respective salt at the temperature at which step (b) is carried out.

**7.** The process according to any one of the preceding claims, wherein the washing buffer in step (b) comprises a salt in a concentration of in the range of 0.7-2.2 M.

**8.** The process according to any one of the preceding claims, wherein the elution buffer in step (c) comprises a salt in an initial concentration of in the range of 0.7-2.2 M.

**9.** The process according to any one of the preceding claims, wherein the elution buffer in step (c) is a gradient buffer with respect to the salt.

**10.** The process according to claim 9, wherein the initial concentration of the salt of the gradient buffer is in the range of 1.7-2.2 M, and the final concentration of the salt of the gradient buffer is in the range of 0.0-1.6 M.

**11.** The process according to any one of the preceding claims, wherein the salt is selected from the group consisting of ammonium acetate, ammonium sulphate, sodium chloride, and sodium acetate.

**Patentansprüche**

**1.** Verfahren zum Reduzieren des Gehalts von Formen von Faktor-VII, welchen ein oder mehrere N-verknüpfte(s) Glycan(e) fehlt (fehlen), in einem Arzneistoff aus einem rekombinant hergestellten aktivierten Faktor-VII-Polypeptid, wobei das Verfahren die folgenden Schritte umfasst:

(a) Inkontaktbringen des Arzneistoffs mit einem hydrophoben Wechselwirkungschromatographie-Material unter Bedingungen, die das Binden eines Teils des Arzneistoffs an das hydrophobe Wechselwirkungschromatographie-Material erleichtern, wobei der Arzneistoff ein Salz, ausgewählt aus der Liste: Ammoniumacetat, Ammoniumsulfat, Ammoniumchlorid, Natriumchlorid, Natriumacetat, Natriumsulfat, Kaliumacetat, Kaliumchlorid und Kaliumsulfat, und/oder ein Zwitterion, ausgewählt aus der Liste: Glycin, Alanin, beta-Alanin, Leucin und Isoleucin, in einer Konzentration im Bereich von 0,0-0,1 M oder im Bereich von 0,5 M bis 85% der Sättigungskonzentration des jeweiligen Salzes bei der Temperatur, bei welcher Schritt (a) durchgeführt wird, umfasst;
(b) wahlweise Waschen des hydrophoben Wechselwirkungschromatographie-Materials mit einem Waschpuffer; und
(c) Eluieren des hydrophoben Wechselwirkungschromatographie-Materials mit einem Elutionspuffer und Sammeln eines gereinigten Arzneistoffs des aktivierten Faktor-VII-Polypeptids als Eluat;

wobei der Gehalt an Formen von Faktor-VII-Polypeptid, welchen ein oder mehrere N-verknüpfte(s) Glycan(e) fehlt (fehlen), im in Schritt (c) gesammelten gereinigten Arzneistoff, wie durch die hier definierten Verfahren bestimmt, verglichen mit dem in Schritt (a) angewendeten Arzneistoff um mindestens 50 % (Gew./Gew.) reduziert wird.

**2.** Verfahren nach Anspruch 1, wobei das hydrophobe Wechselwirkungs-chromatographie-Material aus der Gruppe, bestehend aus mit Butylliganden und/oder Phenylliganden substituierten Harzen ausgewählt wird.

**3.** Verfahren nach einem der vorangehenden Ansprüche, wobei die Arzneistofflast in Schritt (a) im Bereich von mindestens 250 mg Faktor-VII-Polypeptid pro 1 Harz liegt.

**4.** Verfahren nach einem der vorangehenden Ansprüche, wobei der Arzneistoff in Schritt (a) in flüssiger Form vorliegt und eine Ionenstärke von mindestens 50 mS/cm aufweist.

**5.** Verfahren nach einem der vorangehenden Ansprüche, wobei der Arzneistoff von Schritt (a) ein Salz in einer Kon-

EP 1 924 689 B1

zentration im Bereich von 0,5 M bis 85% der Sättigungskonzentration oder 0,7-2,2 M umfasst.

6.  Verfahren nach einem der vorangehenden Ansprüche, wobei der Waschpuffer in Schritt (b) ein Salz, ausgewählt aus der Liste: Ammoniumacetat, Ammoniumsulfat, Ammoniumchlorid, Natriumchlorid, Natriumacetat, Natriumsulfat, Kaliumacetat, Kaliumchlorid und Kaliumsulfat, und/oder ein Zwitterion, ausgewählt aus der Liste: Glycin, Alanin, beta-Alanin, Leucin und Isoleucin, in einer Konzentration im Bereich von 0,0-0,1 M oder im Bereich von 0,5 M bis 85% der Sättigungskonzentration des jeweiligen Salzes bei der Temperatur, bei welcher Schritt (b) durchgeführt wird, umfasst.

7.  Verfahren nach einem der vorangehenden Ansprüche, wobei der Waschpuffer in Schritt (b) ein Salz in einer Konzentration im Bereich von 0,7-2,2 M umfasst.

8.  Verfahren nach einem der vorangehenden Ansprüche, wobei der Elutionspuffer in Schritt (c) ein Salz in einer anfänglichen Konzentration im Bereich von 0,7-2,2 M umfasst.

9.  Verfahren nach einem der vorangehenden Ansprüche, wobei der Elutionspuffer in Schritt (c) ein Gradientenpuffer in Bezug auf das Salz ist.

10. Verfahren nach Anspruch 9, wobei die anfängliche Konzentration des Salzes des Gradientenpuffers im Bereich von 1,7-2,2 M liegt und die Endkonzentration des Salzes des Gradientenpuffers im Bereich von 0,0-1,6 M liegt.

11. Verfahren nach einem der vorangehenden Ansprüche, wobei das Salz aus der Gruppe, bestehend aus Ammoniumacetat, Ammoniumsulfat, Natriumchlorid und Natriumacetat ausgewählt wird.

**Revendications**

1.  Procédé de réduction de la teneur d'une substance médicamenteuse d'un polypeptide du Facteur VII activée et obtenue par recombinaison en formes du Facteur VII auxquelles il manque un ou plusieurs glycanes liés à N, ledit procédé comprenant les étapes de :

    (a) mise en contact de la substance médicamenteuse avec un matériau pour chromatographie d'interaction hydrophobe dans des conditions qui facilitent la liaison d'une portion de ladite substance médicamenteuse audit matériau pour chromatographie d'interaction hydrophobe, ladite substance médicamenteuse comprenant un sel choisi dans la liste consistant en l'acétate d'ammonium, le sulfate d'ammonium, le chlorure d'ammonium, le chlorure de sodium, l'acétate de sodium, le sulfate de sodium, l'acétate de potassium, le chlorure de potassium et le sulfate de potassium, et/ou un zwitterion choisi dans la liste consistant en la glycine, l'alanine, la bêta-alanine, la leucine et l'isoleucine, à une concentration comprise dans la plage de 0,0-0,1 M ou dans la plage de 0,5 M à 85 % de la concentration à saturation, pour le sel considéré, à la température à laquelle est mise en oeuvre l'étape (a) ;
    (b) en option, lavage dudit matériau pour chromatographie d'interaction hydrophobe avec un tampon de lavage ; et
    (c) élution dudit matériau pour chromatographie d'interaction hydrophobe avec un tampon d'élution, et collecte, sous forme d'un éluat, d'une substance médicamenteuse purifiée du polypeptide du Facteur VII activé ;

    dans lequel la teneur de la substance médicamenteuse purifiée recueillie dans l'étape (c) en formes du polypeptide du Facteur VII auxquelles il manque un ou plusieurs glycanes liés à N, telle que déterminée par les méthodes définies dans l'invention, est réduite d'au moins 50 % (p/p) par rapport à la substance médicamenteuse appliquée dans l'étape (a).

2.  Procédé selon la revendication 1, dans lequel le matériau pour chromatographie d'interaction hydrophobe est choisi dans le groupe consistant en les résines substituées par des ligands butyle et/ou des ligands phényle.

3.  Procédé selon l'une quelconque des revendications précédentes, dans lequel la charge de la substance médicamenteuse dans l'étape (a) est comprise dans la plage d'au moins 250 mg du polypeptide du Facteur VII par litre de résine.

4.  Procédé selon l'une quelconque des revendications précédentes, dans lequel la substance médicamenteuse de l'étape (a) se présente sous forme liquide et a une force ionique d'au moins 50 mS/cm.

5. Procédé selon l'une quelconque des revendications précédentes, dans lequel la substance médicamenteuse de l'étape (a) comprend un sel à une concentration comprise dans la plage de 0,5 M à 85 % de la concentration à saturation, ou de 0,7-2,2 M.

6. Procédé selon l'une quelconque des revendications précédentes, dans lequel le tampon de lavage de l'étape (b) comprend un sel choisi dans la liste consistant en l'acétate d'ammonium, le sulfate d'ammonium, le chlorure d'ammonium, le chlorure de sodium, l'acétate de sodium, le sulfate de sodium, l'acétate de potassium, le chlorure de potassium et le sulfate de potassium, et/ou un zwittérion choisi dans la liste consistant en la glycine, l'alanine, la bêta-alanine, la leucine et l'isoleucine, à une concentration comprise dans la plage de 0,0-0,1 M ou dans la plage de 0,5 M à 85 % de la concentration à saturation, pour le sel considéré, à la température à laquelle est mise en oeuvre l'étape (b).

7. Procédé selon l'une quelconque des revendications précédentes, dans lequel le tampon de lavage de l'étape (b) comprend un sel à une concentration comprise dans la plage de 0,7-2,2 M.

8. Procédé selon l'une quelconque des revendications précédentes, dans lequel le tampon d'élution de l'étape (c) comprend un sel à une concentration initiale comprise dans la plage de 0,7-2,2 M.

9. Procédé selon l'une quelconque des revendications précédentes, dans lequel le tampon d'élution de l'étape (c) est un tampon à gradient par rapport au sel.

10. Procédé selon la revendication 9, dans lequel la concentration initiale du sel du tampon à gradient est comprise dans la plage de 1,7-2,2 M, et la concentration finale du sel du tampon à gradient est comprise dans la plage de 0,0-1,6 M.

11. Procédé selon l'une quelconque des revendications précédentes, dans lequel le sel est choisi dans le groupe consistant en l'acétate d'ammonium, le sulfate d'ammonium, le chlorure de sodium et l'acétate de sodium.

**Separation of late eluting impurities using TSK Phenyl 5pw**

Fig. 4/8

Fig. 5/8

**Fig. 6/8**

**Fig. 7/8**

**Fig. 8/8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6777390 B, Baxter
- US 4784950 A
- US 5580560 A
- DK 0200189 W
- WO 0238162 A
- WO 9920767 A
- WO 0158935 A
- WO 0183725 A
- WO 0222776 A
- WO 02077218 A
- WO 0327147 A
- WO 0337932 A
- JP 2001061479 B
- WO 0331464 A
- US 20040043446 A
- US 20040063911 A
- US 20040142856 A
- US 20040137557 A
- US 20040132640 A
- WO 0104287 A
- US 20030165996 A
- WO 0393465 A
- WO 0202764 A
- US 20030211094 A
- US 6806063 B
- US 20030096338 A
- WO 04029091 A
- WO 04083361 A
- WO 04111242 A
- WO 04108763 A
- WO 9215686 A
- US 5997864 A

**Non-patent literature cited in the description**

- Amino acid sequence and posttranslational modifications of human factor VIIa from plasma and transfected baby hamster kidney cells. *Biochemistry,* 04 October 1988, vol. 27 (20), 7785-93
- FVIIa derivatives obtained by autolytic and controlled cathepsin G mediated cleavage. *FEBS Lett.,* 15 February 1993, vol. 317 (3), 245-9
- **PERSSON et al.** *J. Biol. Chem.,* 1997, vol. 272, 19919-19924
- **PERSSON.** *FEBS Letts,* 1997, vol. 413, 359-363
- **LINO et al.** *Arch. Biochem. Biophys.,* 1998, vol. 352, 182-192
- **MOLLERUP et al.** *Biotechnol. Bioeng.,* 1995, vol. 48, 501-505
- **KORNFELT et al.** *Arch. Biochem. Biophys.,* 1999, vol. 363, 43-54
- **WILDGOOSE et al.** *Biochem,* 1990, vol. 29, 3413-3420
- **KAZAMA et al.** *J. Biol. Chem.,* 1995, vol. 270, 66-72
- **HOLST et al.** *Eur. J. Vasc. Endovasc. Surg.,* 1998, vol. 15, 515-520
- **NICOLAISEN et al.** *FEBS Letts.,* 1993, vol. 317, 245-249
- **JURLANDER et al.** *Seminars in Thrombosis and Hemostasis,* 2001, vol. 27 (4
- **MONROE et al.** *Brit. J. Haematol.,* 1997, vol. 99, 542-547
- **WEBER et al.** *Anal. Biochem.,* 1995, vol. 225, 135
- **KLAUSEN et al.** *J. Chromatog.,* 1995, vol. 718, 195
- **MORRIS et al.** Mass Spectrometry of Biological Materials. Marcel Dekker, 1990, 137-167
- **CONBOY et al.** *Biol. Mass Spectrom.,* 1992, vol. 21, 397
- **HELLERQVIST.** *Meth. Enzymol.,* 1990, vol. 193, 554
- **SUTTON et al.** *Anal. Biohcem.,* 1994, vol. 318, 34
- **HARVEY et al.** *Organic Mass Spectrometry,* 1994, vol. 29, 752